# EUROPÄISCHE PATENTANMELDUNG

(11) **EP 4 169 911 A1**
(43) Veröffentlichungstag der Anmeldung: **26.04.2023**
(21) Anmeldenummer: 21204254.3
(22) Anmeldetag: 22.10.2021
(51) Int. Cl.: C07D 311/30

(54) **VERFAHREN ZUR HERSTELLUNG VON MORIN UND MORINDERIVATEN**

(71) Anmelder: Orsatec GmbH, 86399 Bobingen (DE)
(72) Erfinder: Die Erfindernennung liegt noch nicht vor
(74) Vertreter: Stolmár & Partner Patentanwälte PartG mbB

(57) **Zusammenfassung**

Die vorliegende Erfindung betrifft ein Verfahren zur direkten Herstellung von Morinderivaten und hochreinem Morin der Formel (1) 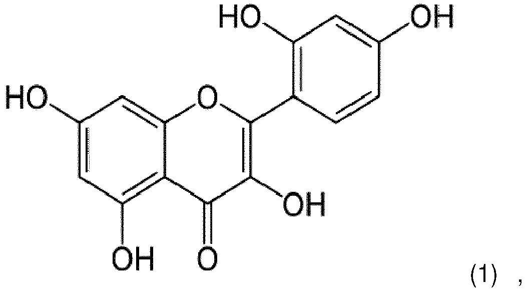 welches die Umsetzung eines Dimethoxyacetophenons mit einem Dimethoxybenzaldehyd zu einem Flavonol umfasst.

## Beschreibung

Der Färbermaulbeerbaum (*Maclura tinctoria* oder auch *Morus tinctoria*) ist ein in Zentralamerika, der Karibik, Ostindien und im tropischen Südamerika heimischer Laubbaum, der eine durchschnittliche Höhe von 20-30 m und eine Stammdicke von 70-80 cm erreichen kann. Das Kernholz des Färbermaulbeerbaumes wird allgemein als Gelbholz bezeichnet und wurde schon in der Präkolumbischen Zeit zum Färben von Textilien verwendet. Die Farbstoffe im Gelbholz sind Maklurin (auch Maclurin oder Moringerbsäure) und das Pentahydroxyflavonol Morin der folgenden Formel I:

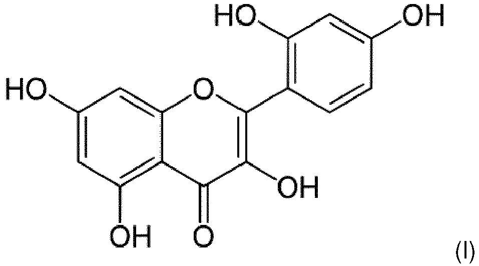

Morin (2,4-Dihydroxyphenyl)-3,5,7-trihydroxy-4*H*-chromen-4-on) gehört zur Gruppe der Flavonole, einer Unterklasse der Flavonoide. Der Name der meist gelben Pflanzenfarbstoffe kommt von lat. flavus = gelb. Morin besitzt fünf Hydroxygruppen und drei zyklische Systeme. Das Grundgerüst der Flavonole mit Nummerierung der Gerüstatome, zusammen mit dem strukturähnlichen Auronen und den offenkettigen Chalkonen, ist der Fig. 1 zu entnehmen.

Morin wirkt antioxidativ und entzündungshemmend und ist kaum toxisch. Somit erfüllt es eine wichtige Voraussetzung für pharmakologische Anwendungen. Die 3-Hydroxylierung bei Morin erhöht verglichen mit den in 3-Stellung unsubstituierten Flavonen dessen antioxidative Wirkung. Aufgrund seines niedrigen p*K*a-Werts von 3,5 (3-OH-Gruppe) liegt Morin unter physiologischen Bedingungen deprotoniert vor und bindet, anders als andere Flavonoide, kaum an negativ geladene DNA oder RNA. Morin (1) komplexiert als exzellenter anionischer Ligand Übergangsmetall- (z. B. 2:1-Komplex mit Zn²⁺)und Lanthanoid-Kationen (z. B. 3:1-Komplex mit La³⁺) unter Deprotonierung der 2'-OH- bzw. 3-OH-Gruppe und findet Anwendung in der fluorimetrischen Analytik diverser Metallionen. Mit Al³⁺ bildet sich ein grün fluoreszierender 3:1-Komplex.

Bis heute wird Morin für den kommerziellen Gebrauch aus dem Extrakt des Gelbholzes isoliert. Allerdings zeigt kommerziell erhältliches Morin (sowohl das natürlich wie auch das synthetisch erhaltene) eine Reinheit von nur etwa 85% mit zumeist der Nebenkomponente Kaempferol (II) als Verunreinigung, dem gegenüber Morin die 2'-Hydroxygruppe fehlt. Darüberhinaus gibt es weitere Verunreinigungen, die üblicherweise ein Gemisch aus Polyphenolen bzw. Flavonoiden sind. Diese Verunreinigungen, die nur extrem aufwändig zu entfernen sind oder gar nicht zu entfernen sind, stören bei vielen Anwendungen, sei es in der Pharmazie oder auch beim Elektroplating oder der Galvanik.

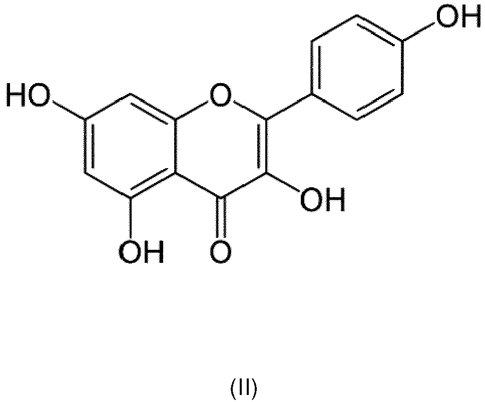

Im Labormaßstab werden im Stand der Technik zwei Synthesewege beschrieben. Obwohl die Struktur von Morin bereits 1896 durch Perkin aufgeklärt wurde und umfangreiche Literatur zur Synthese von Flavonoiden allgemein existiert, wurde Morin erst 2013 synthetisiert, wobei eine Allan-Robinson-Kondensation ausgehend von 2-Methoxy-1-(2,4,6-trihydroxyphenyl)ethan-1-on und 2,4-Dimethoxybenzoylchlorid genutzt wird (CN 103342690 A). Weiterhin wurde eine ähnliche Route zu Morin beschrieben, die über das Chalkon (*E*)-3-(2,4-Dimethoxyphenyl)-1-(2-hydroxy-4,6-dimethoxyphenyl)prop-2-en-1-on (8) verläuft (CN 105985306). Dabei wird die Aldolkondensation des Acetophenon-Derivats 1-(2-Hydroxy-4,6-dimethoxyphenyl)ethan-1-on mit 2,4-Dimethoxybenzaldehyd genutzt. Schließlich wurde ein Syntheseweg beschrieben, der ohne die Methylierung der phenolischen Hydroxygruppen auskommen soll (B. Vyas, M. Singh, M. Kaur, O. Silakari, M. S. Bahia, B. Singh, Med. Chem. Res. 2016, 25, 609-626).

In der WO 2019/101353 A1 wird ein Verfahren zur direkten Synthese von hochreinem Morin und hochreinen Morinderivaten beschrieben, welche die vorstehend genannten Verunreinigungen nicht mehr enthalten. Dieses Verfahren beruht auf leicht erhältlichen und kostengünstigen Ausgangsstoffen und umfasst eine Mehrstufensynthese. Aber auch hier besteht nocht Verbesserungspotenzial hinsichtlich einer möglichen Reduzierung der Verfahrensschritte (Reduktion der Synthesestufen). Da sich jeder zusätzliche Verfahrensschritt negativ auf die Ausbeute am gewünschten Endprodukt auswirkt, umfasst das Verbesserungspotenzial nicht nur eine Verfahrensvereinfachung sondern gleichzeitig eine mögliche Ausbeutesteigerung und damit eine Reduktion der Herstellungskosten des Endprodukts.

Für die Zwecke einer möglichst detaillierten Offenbarung der vorliegenden Erfindung und zu deren einfachem Verständnis werden die Beschreibung und die Ansprüche der WO 2019/101353 A1 explizit in die hier vorliegende Beschreibung mit einbezogen. Dies gilt insbesondere für das dort offenbarte Syntheseschema. Bei der vorliegenden Erfindung sind gegenüber der WO-Schrift die beiden ersten Reaktionsschritte Acetylierung und Alkylierung getauscht.

Somit bestand die Aufgabe der vorliegenden Erfindung in der Bereitstellung eines verbesserten Syntheseverfahrens für reines und hochreines Morin und reine und hochreine Morinderivate.

Die Aufgabe wird gelöst durch ein Verfahren zur Herstellung einer Verbindung mit der folgenden Formel (1), 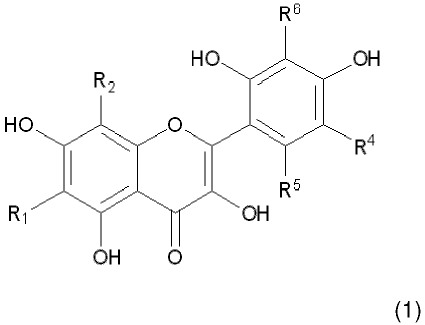 umfassend die Schritte:
i) Acetylierung einer Verbindung der folgenden Formel (3) 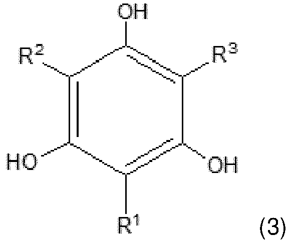 zur Bildung des Acetophenons der Formel (4), 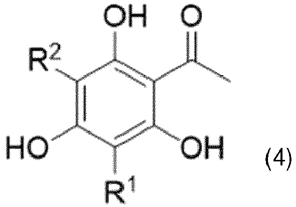
ii) alkylierung des Acetophenons der Formel (4) dabei wird die Verbindung der Formel (6) gebildet 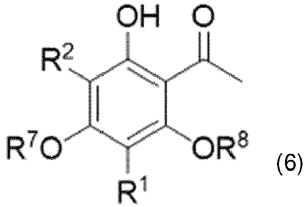
iii) Umsetzen des Acetophenons der Formel (6) mit einer Verbindung der folgenden Formel (7) 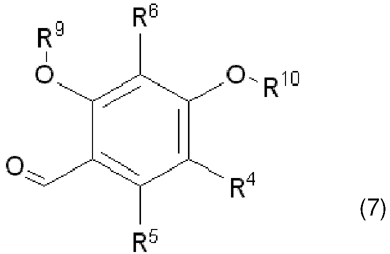 zur Bildung eines Flavonols der folgenden Formel (8), 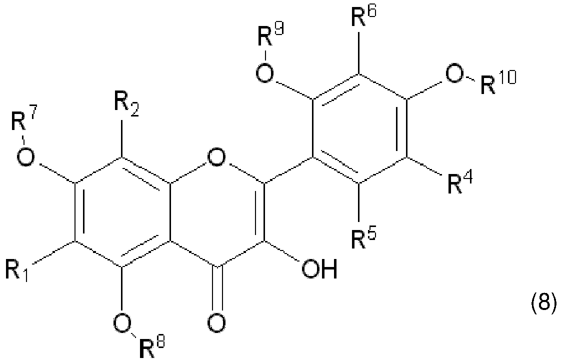 und
iv) Entfernung der an Sauerstoff gebundenen Schutzgruppen durch Dealkylierung, insbesondere Demethylierung, des Flavonols der Formel (8) zum Erhalt der Verbindung der Formel (1),
wobei
R¹, R², R³, R⁴, R⁵ und R⁶ unabhängig voneinander ein verzweigtes oder unverzweigtes C₁-C₈₋Alkyl, NO₂, SO₃H, Cl, Br, NX₂, wobei X ein Ethyl- oder Methylrest oder Wasserstoff ist, CF₃ oder Wasserstoff sind, bevorzugt C₁-C₅-Alkyl oder Wasserstoff, insbesondere bevorzugt C₁-C₃-Alkyl oder Wasserstoff, und
R⁷, R⁸, R⁹ und R¹⁰ unabhängig voneinander eine Methyl-, Ethyl-, *tert*.-Butyl-, Benzyl-, Methoxymethyl-, p-Methoxybenzyl-, Benzyloxymethyl-, Triphenylmethyl-, Tetrahydropyranyl- , Allyl-, Trimethylsilyl-, Triethylsilyl-, Triisopropylsilyl- Gruppe sind und insbesondere R⁷, R⁸, R⁹ und R¹⁰ eine Methylgruppe bedeuten.

Das Verfahren beruht dabei auf leicht erhältlichen Ausgangsstoffen und bietet eine gegenüber dem Stand der Technik deutlich gesteigerte Ausbeute für das gewünschte Produkt. Zudem wird das Produkt in hoher Reinheit gewonnen, welches somit eine höhere Aktivität als natürlich gewonnenes Morin zeigt.

### Das Verfahren kann zusätzlich oder alternativ dazu einen Schritt

### (i-a) Methylierung einer Verbindung der folgenden Formel (3)

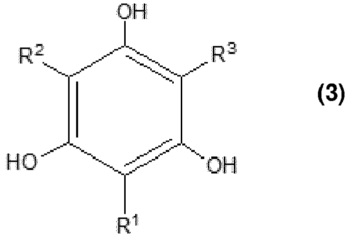

**unter sauren Bedingungen zur Bildung einer Verbindung der Formel (6) nach erfolgter Acetylierung umfassen, wobei R¹ bis R⁸ wie oben definiert sind.**

**Hierdurch können Morin bzw. Morinderivate aus weiteren, leicht erhältlichen Ausgangsstoffen synthetisiert werden, z.B. dem kommerziell erhältlichen Trimethoxybenzol, das direkt eingesetzt werden kann.**

In einer bevorzugten Ausführungsform ist R³ Wasserstoff. In einer besonders bevorzugten Ausführungsform sind R¹, R², R³, R⁴, R⁵ und R⁶ Wasserstoff. Somit wird durch das erfindungsgemäße Verfahren besonders bevorzugt hochreines Morin der Formel I erhalten:

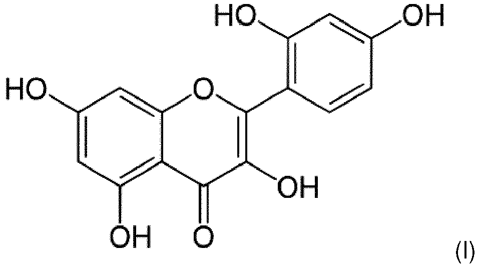

In Schritt i) kann die Acetylierung mit Hilfe von Acetylchlorid in Anwesenheit einer Lewis-Säure, Dichlormethan und Nitromethan erfolgen. Lewis-Säuren können hierbei bevorzugt Bortrichlorid, BBr₃ oder AlCl₃ oder Mischungen der vorgenannten sein, ganz besonders ist AlCl₃ oder Bortrichlorid bevorzugt.

In Schritt (ii) wird die Alkylierung, bevorzugt Methylierung, in Aceton über Dimethylsulfoxid, Dimethylsulfat, Diazomethan, Methyltriflat, Methyliodid, Methyl-p-toluolsulfonat oder Dimethylcarbonat durchgeführt. Bevorzugt könnnen Methyl-p-toluolsulfonat sowie Dimethylsulfat verwendet werden.

Schritt iii), Umsetzung der Verbindung (6) mit der Verbindung (7) zu einem Flavonol der Formel (8), repräsentiert den Kern der vorliegenden Erfindung. Die Umsetzung erfolgt in einem basischen Milieu bei gleichzeitig vorliegenden oxidierenden Reaktionsbedingungen. Die Einstellung der basischen Reaktionsbedingungen erfolgt durch eine Reaktionsführung in einer wässrigen Pyrrolidin-Lösung. Pyrrolidin ist als sekundäres, heterocyclisches Amin mit Wasser vollständig und mit den meisten organischen Lösungsmitteln gut bis sehr gut mischbar. Dabei verhält sich Pyrrolidin als starke Base. Weiterhin erfolgt die Umsetzung bei erhöhter Temperatur (bevorzugt bei ca. 50 °C) über einen längeren Zeitraum (z.B. über 48 h). Um die oxidierenden Reaktionsbedingungen sicherzustellen, erfolgt die Umsetzung in einer Sauerstoffatmosphäre. Zur Steigerung des Oxidationspotenzials wird bevorzugt die Bildung von Singulett-Sauerstoff während der Reaktion unterstützt. Dies geschieht wiederum bevorzugt durch eine sogenannte Triplett-Triplett-Energieübertragung mit Hilfe von sog. Triplettgeneratoren in einer photochemischen Reaktion. Als Triplettgeneratoren sind insbesondere Farbstoffe wie z.B. Methylenblau oder Eosin Y geeignet. Chemisch kann Singulett-Sauerstoff aus Peroxiden, wie z.B. Wasserstoffperoxid, mit Natriumhypochlorit (NaClO) erzeugt werden.

Erfindungsgemäß ist somit jedes Verfahren zur Herstellung von Morinderivaten und Morin als vorteilhaft zu bewerten, welches den Reaktionsschritt iii) umfasst, und zwar unabhängig von den weiteren Reaktionsschritten (vor- oder nachgelagert). Dies gilt somit auch für das Verfahren gemäß WO 2019/101353 A1, welches erfindungsgemäß bzgl. Einfachheit der Verfahrensführung und Ausbeute deutlich verbessert wird.

Für den Reaktionsschritt iii) können neben der Base Pyrrolidin z.B. auch Piperidin, Morpholin oder Diethylamin eingesetzt werden.

Die Molverhältnisse der Base zu den Edukten liegen im Berreich 2― 10 Äquivalenten. Dabei wird der Bereich von 5 ― 10 Äquivalenten bevorzugt

Bevorzugt sollte die Reaktion iii) in einem basischen Milieu stattfinden. Insbesondere im pH-Bereich 12 -14

Im Allgemeinen läuft diese Reaktion mit einer Dauer von 12 bis 72 Stunden bei einer Temperatur von 25 °C bis 60 °C. Bevorzugt wird die Reaktion bei einer Reaktiosdauer von 24 bis 48 Stunden und im Temperaturbereich von 40 °C bis 50 °C durchgeführt.

Weitere Beispiele für Triplettgeneratoren sind die Verbindungsklasse der Xanthenfarbstoffe: (z.B. Eosin B, Eosin Y, Fluorescein) und die Verbindungsklasse der Triphenylmethylfarbstoffe: (z.B. Acid Red 94, Fuchsin, Fluorescein).

Die Bestrahlung des Reaktionsmediums erfolgt bevorzugt im Wellenlängenberreich zwischen 350 nm und 580 nm, speziell bei der Nutzung der genannten Triplettgeneratoren zwischen 500 nm und 580 nm. In diesem Bereich liegen die jeweiligen Absorbtionsmaxima der genannten Triplletgeneratoren.

Die in dem erfindungsgemäßen Reaktionsschema auftretenden Alkylierungen und Dealkylierungen, bevorzugt in Form von Methylierungen und Demethylierungen, werden nach dem Fachmann bekannten Lehrbuchmethoden (z.B. gemäß dem Standardwerk "Organikum" /Organisch chemisches Grundpraktikum) durchgeführt und dienen insbesondere zur Einführung und zur Entfernung von Schutzgruppen, wie z.B. MOM (Methoxymethylschutzgruppe), Allyl, Tr (Triphenylmethylschutzgruppe), Bn (Benzylschutzgruppe), TMS (Trimethylsilylschutzgruppe) oder TES (Triethylsilylschutzgruppe).

So kann beim erfindungsgemäßen Verfahren, insbesondere bei der Verwendung der Schutzgruppe MOM, bei der Durchführung des Reaktionsschrittes iv) (Überführung eines Flavonols (8) in eine Verbindung der Formel (1)) auf den Einsatz von HBr/H₂O (48 %; vgl. Beispiel 5 der WO 2019/101353 A1) verzichtet werden. Wie in den nachfolgenden Ausführungsbeispielen gezeigt, wird bei der Aufarbeitung des Pyrrolidin-Ansatzes ohnehin mit Säure (bevorzugt HCl) neutralisiert und mit einem leichten Säureüberschuss die MOM-Schutzgruppe durch Hydrolyse entfernt.

Durch das erfindungsgemäße Verfahren kann einfach und vorteilhaft eine kaempferolfreie Verbindung mit der Formel (1) bereitgestellt werden. In einer besonders bevorzugten Ausführungsform sind in der Verbindung der Formel (1) R¹, R², R³, R⁴, R⁵ und R⁶ Wasserstoff, d.h die Verbindung ist kaempferolfreies Morin der Formel (I). Das erfindungsgemäße Morin zeichnet sich durch eine sehr hohe Reinheit aus; bevorzugt beträgt die Reinheit mehr als 85%, 90% oder ganz besonders bevorzugt mehr als 95%, in besonders bevorzugten Ausführungsformen wird eine Reinheit von 99% erzielt. Reinheit bedeutet vorliegend, dass Polyphenole bzw. Flavonoide nur in den vorgenannten Mengen nachweisbar sind. Insbesondere ist wie schon gesagt das Morin gemäß der vorliegenden Erfindung frei von Kaempferol Verunreinigungen, d.h. frei von Kaempferol der Formel (II) und/oder seinen Derivaten. Vorliegend bedeutet der Ausdruck "frei von", dass im Rahmen der üblichen Meßgenauigkeit Kaempferol bzw. dessen Derivate in einem Anteil von weniger als 0,5 %, in spezifischen Ausgestaltungen von weniger als 0,3 % vorhanden sind. In besonders bevorzugten Ausführungsformen sind Kaempferol bzw. dessen Derivate im Rahmen der Meßgenauigkeit nicht nachweisbar..Das hochreine Morin erhältlich gemäß der vorliegenden Erfindung kann z.B. direkt ohne weitere Aufreinigung bei der elektrolytischen Zinn- oder Zinn/Bleiabscheidung (z.B. gemäß der EP 810303 A1 oder der DE 19623274 A1) eingesetzt werden. Es wurde gefunden, dass die Aktivität des Morins in der Sn und Sn/Pb Abscheidung nicht linear mit dem Gehalt an Morin in dem bislang verwendeten Naturprodukt korreliert, was auf die nicht entfernbaren Verunreinigungen, insbesondere Kaempferol und Polyphenole und Flavonoide, zurückzuführen ist. Man nimmt an, dass natürliche, technisch bislang nicht entfernbare Verunreinigungen die Aktivität des Morins absenken. Das reine Syntheseprodukt gemäß der vorliegenden Erfindung enthält diese natürlichen Verunreinigungen nicht und zeigt daher eine gegenüber dem Naturprodukt um 22,35% verbesserte Aktivität. Verbesserte Aktivität im Sinne der vorliegenden Erfindung bedeutet dabei eine um diesen Prozentsatz vergrößerte Bedeckung eines Bleches mit Sn oder Sn/Pb in einer galvanotechnischen sogenannten Hull-Zelle, wie es in der EP 810303 A1 detailliert beschrieben wird. Darüberhinaus ist die Porosität der mittels des erfindungsgemäßen Morins erhaltenen Zinn bzw. Zinn/Bleischicht (gemessen mittels Feuchtetransport FSP, Diss. H. Künzel, Univ. Stuttgart 1994) um ca. 35% geringer als bei dem Vergleichsbeispiel, das mittels des natürlichen Morins hergestellt wurde. Dabei wurde festgestellt, dass unabhängig von der vorliegend getesteten Bezugsquelle (Sigma-Aldrich, Adooq Bioscience, Aurantika, Fisher-Scientific) des natürlichen Morins, die verbesserten Eigenschaften immer in der gleichen Größenordnung (im Rahmen der üblichen Messtoleranzen) sind.

Da bei dem erfindungsgemäßen Verfahren die Verbindungen (6) und (7) in einem einstufigen Prozess zu dem Flavonol (8) umgesetzt werden, unter Wegfall der Zwischenstufe eines Chalkons (8) gemäß dem **Reaktionsschema aus der** WO 2019/101353 A1**, kann die Ausbeute an den gewünschten Endprodukten gegenüber dieser bekannten Synthesroute deutlich gesteigert werden.**

Für die Reaktionsschritte Verbindung (6) umsetzen mit Verbindung (7) zu dem Chalkon (8) und für die Umsetzung des gebildeten Chalkons (8) zu dem Flavonol (9) werden in der WO 2019/101353 A1 Ausbeuten von 70% und 38% genannt, was einer Gesamtausbeute aus diesen beiden Schritten von 26,6% entspricht. Bei der erfindungsgemäß in einem Schritt ablaufenden Umsetzung der Verbindungen (6) und (7) zu dem Flavonol (in der vorliegenden Beschreibung mit (8) bezeichnet) ergibt sich hingegen eine Ausbeute von 60% und damit eine Steigerung um 125% gegenüber dem Stand der Technik.

Somit umfasst die vorliegende Verbindung allgemein ein Verfahren zur Herstellung von Morin und Morinderivaten, welches dadurch gekennzeichnet ist, dass eine Verbindung (6) mit einer Verbindung (7) in einem Schritt oder in einer Synthesestufe, unter Umgehung eines Chalkons, zu einem Flavonol (8) umgesetzt wird. Dieser Schritt vereinfacht die Komplexität des Verfahrens, erhöht die Ausbeute an dem Flavonol (8) und damit letztlich auch an dem gewünschten Endprodukt (1) und führt somit insgesamt zu einer Senkung der Herstellungskosten für Morin und Morinderivate (1).

Die Erfindung wird nachstehend anhand von Beispielen erläutert, die jedoch nicht als beschränkend verstanden werden sollen, vielmehr handelt es sich um erfindungsgemäß bevorzugte Ausführungsformen..

### Beispiel 1 Herstellung von 2,4,6 Trihydroxyacetophenon

Unter Argonatmosphäre wurde Phloroglucinol (3) (10.0 g, 79.3 mmol, 1.0 Äq.) zu einer Mischung aus Dichlormethan 187 ml und Nitromethan 52.8 ml gegeben. Es wurde langsam Aluminiumchlorid (28.5 g, 213 mmol, 2.9 Äq.) zugegeben. Die Reaktionslösung wurde auf 43 °C erhitzt und langsam Acytylchlorid (6.5 g, 83.3 mmol, 1.05 Äq.) zugetropft. Die Dauer der Zugabe betrug 3 Stunden. Nach der Zugabe wurde die Reaktionslösung für 1 Stunde bei 43 °C bei Rückfluss gerührt. Im Anschluss wurde das Dichlormethan aus der Reaktionslösung bis zu einer Lösungstemperatur von 85 °C rausdestilliert. Die Reaktionslösung wurde auf Raumtemperatur abgekühlt und dest. Wasser wurde langsam zugegeben. Dabei darf die Temperatur der Reaktionslösung nicht höher als 40 °C werden. Die entstandene Suspension wurde abfiltriert und getrocknet. Der trockene Filterkuchen wurde in Ethylacetat gelöst und das ungelöste Salz abfiltriert. Das Lösungsmittel wurde entfernt und das Produkt (10.0 g, 59,5 mmol, 75 %) als ein hellbrauner Feststoff erhalten.
DC [Petrolether/Ethylacetat (2:1)]: *R*_{f} = 0.20. ¹H-NMR (400 MHz, DMSO-*d*₆): δ = 12.22 (s, 2H, OH), 10.36 (s, 1H, OH), 5.80 (s, 2H, C_{Ar}-H), 2,54 (s, 3H, CH₃). ¹³C-NMR (100 MHz, DMSO-*d*₆): δ = 202.5 (C=O), 164.8 (C_{Ph}-4-OH), 164.4 (2C, Cₚₕ-2-OH, Cₚₕ-6-OH), 104.1 (Cₚₕ-1), 94.6 (2C, Cₚₕ-3, Cₚₕ-5), 32.4 (CH₃).
MS (ESI): m/z (%) = 167 [M - H]- (100)

### Beispiel 2: Herstellung von 2-Hydroxy-4,6-dimethoxyacetophenon

Unter Argonatmosphäre wurde 2,4,6-Trihydroxyacetophenon (20.0 g, 119 mmol, 1.0 Äq.) in 400 ml Aceton gelöst. Zu der Lösung wurden Natriumcarbonat (132 g, 950 mmol, 8.0 Äq.) sowie *p*-Toluolsulfonsäuremethylester (89 g, 480 mmol, 4.0 Äq.) zugegeben. Die Reaktionslösung wurde für 11 Stunden bei 72 °C bei Rückfluss gerührt. Im Anschluss wurde das Aceton rausdestilliert und 200 ml dest. Wasser zugegeben. Die Reaktionslösung für 4 Stunden bei 85°C gerührt. Die Reaktrionslösung wurde auf Raumtemperatur abgekühlt und 2M Salzsäure wurde, bis der pH Wert von 9-10 erreicht wurde, zugegeben. Die Suspension wurde filtriert und das Produkt (18.7 g, 95.1 mmol, 80%) wurde als beiger Feststoff erhalten.

DC [Hexan/Ethylacetat (2:1)]: *R*_{f} = 0.57. Schmp.: 79-82 °C. ¹H-NMR (400 MHz, CDCl₃): δ = 14.03 (s, 1H, OH), 6.04 (d, *J* = 2.4 Hz, 1H, 5-CH), 5.91 (d, *J* = 2.4 Hz, 1H, 3-CH), 3.84 (s, 3H, *o*-Ph-OCH₃), 3.81 (s, 3H, *p*-Ph-OCH₃), 2.60 (s, 3H, O=CCH₃). ¹³C-NMR (100 MHz, CDCl₃): δ = 203.2 (C=O), 167.7 (*p*-C_{Ph}-OCH₃), 166.2 (*o*-C_{Ph}-OCH₃), 163.0 (*o*-C_{Ph}-OH), 106.1 (C_{Ph}-C=O), 93.6 (C-5), 90.8 (C-3), 55.6 (2C, OCH₃), 33.0 (CH₃).
MS (ESI): m/z (%) = 197 [M + H]+ (60), 219 [M + Na]+ (100)

### Beispiel 3 a (ohneTriplettgenerator)

### Herstellung von 2-(2,4-Dimethoxyphenyl)-3-hydroxy-5,7-dimethoxy-4H-chromen-4-on (8)

2-Hydroxy-4,6-dimethoxyacetophenon (10.0 g, 51.0 mmol, 1.0 Äq.), 2,4-Dimethoxybenzaldehyd (9.0 g, 54.0 mmol, 1.05 Äq.) sowie Pyrrolidin (37 g, 510 mmol, 10.0 Äq.) wurden in 810 ml dest. Wasser und 30 ml Ethanol gegeben. Unter Rühren wird diese Mischung unter Sauerstoffatmosphäre auf 50 °C erhitzt. Die Mischung wird mit Licht mit einem Tageslicht-Spektrum bestrahlt und 48 Stunden bei 50 °C gerührt. Die Bestrahlung erfolgt während der gesammten Laufzeit der Reaktion. Anschließend wird die Reaktionsmischung auf Raumtemperatur abgekühlt und mit 2M HCl auf einen pH-Wert von pH 2-3 eingestellt und weitere 2-3 Stunden bei Raumtemperatur gerührt. Der Inhalt des Reaktionsgefäßes wird filtriert und der Filterkuchen mit dest. Wasser bis zum pH Wert 6-7 gewaschen. Der so erhaltene Filterkuchen wird anschließend im Trockenschrank bei Vakuum und 50 °C über Nacht getrocknet und in n-Butanol umkristallisiert. Das Produkt (10.9 g, 30.5 mmol, 60 %) wurde als weiss-gelber Feststoff erhalten.

¹H-NMR (400 MHz, CDCl₃): δ = 7.49 (d, *J* = 8.4 Hz, 1H, 6'-H), 6.59 (dd, *J* = 8.4, 2.3 Hz, 1H, 5'-H), 6.56 (d, *J* = 2.3 Hz, 1H, 3'-H), 6.46 (d, *J* = 2.3 Hz, 1H, 8-H), 6.33 (d, *J* = 2.3 Hz, 1H, 6-H), 3.95 (s, 3H, 4'-OCH₃), 3.85 (s, 6H, 2'-OCH₃, 7-OCH₃), 3.84 (s, 3H, 5-OCH₃ ), ¹³C-NMR (100 MHz, CDCl₃): δ = 172.1 (C=0), 164.2 (C-7), 162.8 (C-4'), 160,7 (C-5), 159.7 (8C-C-1O), 159.0 (C-2'), 143.0 (C-2), 138.7 (C-3), 132.0 (C-6'), 112.5 (C-1'), 106.9 (4C-C-5C), 104.9 (C-5'), 99.3 (C-3'), 95.6 (C-6), 92.6 (C-8), 56.4 (4'-OCH₃), 55.9 (5-OCH₃), 55.8 (2'-OCH₃), 55.6 (7-OCH₃)
MS (ESI): *m*/*z* (%) = 359 [M+ H]+ (35), 381 [M + Na]⁺ (41), 739 [2M + Na]⁺ (100).

### Beispiel 3 b (mit Triplettgenerator)

### Herstellung von 2-(2,4-Dimethoxyphenyl)-3-hydroxy-5,7-dimethoxy-4H-chromen-4-on (8)

2-Hydroxy-4,6-dimethoxyacetophenon (10.0 g, 51.0 mmol, 1.0 Äq.), 2,4-Dimethoxybenzaldehyd (9.0 g, 54.0 mmol, 1.05 Äq.), Pyrrolidin (37 g, 510 mmol, 10.0 Äq.) sowie 200 mg EOSIN Y wurden in 810 ml dest. Wasser und 30 ml Ethanol gegeben. Unter Rühren wird diese Mischung unter Sauerstoffatmosphäre auf 50 °C erhitzt. Die Mischung wird mit Licht der Wellenlängen 520 nm ― 560 nm bestrahlt und 48 Stunden bei 50 °C gerührt. Die Bestrahlung erfolgt während der gesammten Laufzeit der Reaktion. Anschließend wird die Reaktionsmischung auf Raumtemperatur abgekühlt und mit 2M HCl auf einen pH-Wert von pH 2-3 eingestellt und weitere 2-3 Stunden bei Raumtemperatur gerührt. Der Inhalt des Reaktionsgefäßes wird filtriert und der Filterkuchen mit dest. Wasser bis zum pH Wert 6-7 gewaschen. Der so erhaltene Filterkuchen wird anschließend im Trockenschrank bei Vakuum und 50 °C über Nacht getrocknet und in n-Butanol umkristallisiert. Das Produkt (11.8 g, 33.1 mmol, 65 %) wurde als weiss-gelber Feststoff erhalten.

¹H-NMR (400 MHz, CDCl₃): δ = 7.49 (d, *J* = 8.4 Hz, 1H, 6'-H), 6.59 (dd, *J* = 8.4, 2.3 Hz, 1H, 5'-H), 6.56 (d, *J* = 2.3 Hz, 1H, 3'-H), 6.46 (d, *J* = 2.3 Hz, 1H, 8-H), 6.33 (d, *J* = 2.3 Hz, 1H, 6-H), 3.95 (s, 3H, 4'-OCH₃), 3.85 (s, 6H, 2'-OCH₃, 7-OCH₃), 3.84 (s, 3H, 5-OCH₃ ),

¹³C-NMR (100 MHz, CDCl₃): δ = 172.1 (C=0), 164.2 (C-7), 162.8 (C-4'), 160,7 (C-5), 159.7 (8C-C-1O), 159.0 (C-2'), 143.0 (C-2), 138.7 (C-3), 132.0 (C-6'), 112.5 (C-1'), 106.9 (4C-C-5C), 104.9 (C-5'), 99.3 (C-3'), 95.6 (C-6), 92.6 (C-8), 56.4 (4'-OCH₃), 55.9 (5-OCH₃), 55.8 (2'-OCH₃), 55.6 (7-OCH₃)

MS (ESI): *m*/*z* (%) = 359 [M+ H]+ (41), 381 [M + Na]⁺ (50), 739 [2M + Na]⁺ (100).

### Beispiel 3 c (ohneTriplettgenerator)

### Herstellung von 2-(2,4-Dimethoxyphenyl)-3-hydroxy-5,7-dimethoxy-4H-chromen-4-on (8)

2-Hydroxy-4,6-dimethoxyacetophenon (1.0 g, 5.1 mmol, 1.0 Äq.), 2,4-Dimethoxybenzaldehyd (932 mg, 5.61 mmol, 1.1 Äq.) sowie Pyrrolidin (3.6 g, 51 mmol, 10.0 Äq.) wurden in 34 ml dest.

Wasser und 2.9 ml Methanol gegeben. Unter Rühren wird diese Mischung unter Sauerstoffatmosphäre auf 50 °C erhitzt. Die Mischung wird mit Licht mit einem Tageslicht-Spektrum bestrahlt und 24 Stunden bei 50 °C gerührt. Die Bestrahlung erfolgt während der gesammten Laufzeit der Reaktion. Anschließend wird die Reaktionsmischung auf Raumtemperatur abgekühlt und mit 2M HCl auf einen pH-Wert von pH 2-3 eingestellt und weitere 2-3 Stunden bei Raumtemperatur gerührt. Der Inhalt des Reaktionsgefäßes wird filtriert und der Filterkuchen mit dest. Wasser bis zum pH Wert 6-7 gewaschen. Der so erhaltene Filterkuchen wird anschließend im Trockenschrank bei Vakuum und 50 °C über Nacht getrocknet und in n-Butanol umkristallisiert. Das Produkt (1.46 g, 4.08 mmol, 80 %) wurde als weiss-gelber Feststoff erhalten.

¹H-NMR (400 MHz, CDCl₃): δ = 7.49 (d, *J* = 8.4 Hz, 1H, 6'-H), 6.59 (dd, *J* = 8.4, 2.3 Hz, 1H, 5'-H), 6.56 (d, *J* = 2.3 Hz, 1H, 3'-H), 6.46 (d, *J* = 2.3 Hz, 1H, 8-H), 6.33 (d, *J* = 2.3 Hz, 1H, 6-H), 3.95 (s, 3H, 4'-OCH₃), 3.85 (s, 6H, 2'-OCH₃, 7-OCH₃), 3.84 (s, 3H, 5-OCH₃ ),

¹³C-NMR (100 MHz, CDCl₃): δ = 172.1 (C=0), 164.2 (C-7), 162.8 (C-4'), 160,7 (C-5), 159.7 (8C-C-1O), 159.0 (C-2'), 143.0 (C-2), 138.7 (C-3), 132.0 (C-6'), 112.5 (C-1'), 106.9 (4C-C-5C), 104.9 (C-5'), 99.3 (C-3'), 95.6 (C-6), 92.6 (C-8), 56.4 (4'-OCH₃), 55.9 (5-OCH₃), 55.8 (2'-OCH₃), 55.6 (7-OCH₃)

MS (ESI): *m*/*z* (%) = 359 [M+ H]+ (35), 381 [M + Na]⁺ (41), 739 [2M + Na]⁺ (100).

### Beispiel 3 d (mit Triplettgenerator)

### Herstellung von 2-(2,4-Dimethoxyphenyl)-3-hydroxy-5,7-dimethoxy-4H-chromen-4-on (8)

2-Hydroxy-4,6-dimethoxyacetophenon (500 mg, 2.55 mmol, 1.0 Äq.), 2,4-Dimethoxybenzaldehyd (466 mg, 2.80 mmol, 1.1 Äq.), Pyrrolidin (1.81 g, 25.4 mmol, 10.0 Äq.) sowie 15 mg Methylenblau wurden in 17 ml dest. Wasser und 1.5 ml Methanol gegeben. Unter Rühren wird diese Mischung unter Sauerstoffatmosphäre auf 50 °C erhitzt. Die Mischung wird mit Licht der Wellenlängen 530 nm ― 700 nm bestrahlt und 24 Stunden bei 50 °C gerührt. Die Bestrahlung erfolgt während der gesammten Laufzeit der Reaktion. Anschließend wird die Reaktionsmischung auf Raumtemperatur abgekühlt und mit 2M HCl auf einen pH-Wert von pH 2-3 eingestellt und weitere 2-3 Stunden bei Raumtemperatur gerührt. Der Inhalt des Reaktionsgefäßes wird filtriert und der Filterkuchen mit dest. Wasser bis zum pH Wert 6-7 gewaschen. Der so erhaltene Filterkuchen wird anschließend im Trockenschrank bei Vakuum und 50 °C über Nacht getrocknet und in n-Butanol umkristallisiert. Das Produkt (776 mg, 2.17 mmol, 85 %) wurde als weiss-gelber Feststoff erhalten.

¹H-NMR (400 MHz, CDCl₃): δ = 7.49 (d, *J* = 8.4 Hz, 1H, 6'-H), 6.59 (dd, *J* = 8.4, 2.3 Hz, 1H, 5'-H), 6.56 (d, *J* = 2.3 Hz, 1H, 3'-H), 6.46 (d, *J* = 2.3 Hz, 1H, 8-H), 6.33 (d, *J* = 2.3 Hz, 1H, 6-H), 3.95 (s, 3H, 4'-OCH₃), 3.85 (s, 6H, 2'-OCH₃, 7-OCH₃), 3.84 (s, 3H, 5-OCH₃ ),

¹³C-NMR (100 MHz, CDCl₃): δ = 172.1 (C=0), 164.2 (C-7), 162.8 (C-4'), 160,7 (C-5), 159.7 (8C-C-1O), 159.0 (C-2'), 143.0 (C-2), 138.7 (C-3), 132.0 (C-6'), 112.5 (C-1'), 106.9 (4C-C-5C), 104.9 (C-5'), 99.3 (C-3'), 95.6 (C-6), 92.6 (C-8), 56.4 (4'-OCH₃), 55.9 (5-OCH₃), 55.8 (2'-OCH₃), 55.6 (7-OCH₃)

MS (ESI): *m*/*z* (%) = 359 [M+ H]+ (41), 381 [M + Na]⁺ (50), 739 [2M + Na]⁺ (100).

### Beispiel 4

### Herstellung von 2-(2,4-Dihydroxyphenyl)-3,5,7-trihydroxy-4H-chromen-4-on (I, Morin)

Unter Argotatmosphäre wurde das Flavonol (8) (1.0 g, 2.79 mmol, 1.0 Äq.) in 7 ml 100 % Methansulfonsäure gelöst und HBr (48 % in H₂O) (21.2 g, 126 mmol, 15 Äq.) zugegeben. Anschließend wurde für 48 Stunden bei 120 °C gerührt. Im Anschluss wurde die Reaktionsösung auf Raumtemperatur abgekühlt und Wasser zugegeben. Der entstandene Niederschlag wurde abfiltriert und mit dest. Wasser gewaschen. Nach Trocknung im Trockenschrank bei Vakuum und 50 °C wurde Morin (1) (830 mg, 2,74 mmol, 98%) als hellbrauner Feststoff erhalten.

1H-NMR (400 MHz, DMSO-d6): δ = 12.61 (s, 1H, 5-COH), 10.72 (s, 1H, COH), 9.73 (s, 2H, 4'-COH, COH), 8.83 (s, 1H, COH), 7.23 (d, *J* = 8.4 Hz, 1H, 6'-H), 6.40 (d, *J* = 2.3 Hz, 1H, 3'-H), 6.35 (dd, *J* = 8.5, 2.3 Hz, 1H, 5'-H), 6.30 (d, *J* = 2.1 Hz, 1H, 8-H), 6.18 (d, *J* = 2.1 Hz, 1H, 6-H).

13C-NMR (101 MHz, DMSO-d6): δ = 176.2 (C-4), 163.7 (C-7), 160.9 (C-5), 160.4 (C-4'), 156.8 (2C, C-2', C-8a), 149.0 (C-2), 136.2 (C-3), 131.7 (C-6'), 109.2 (C-1'), 106.8 (C-5'), 103.6 (C4a), 103.0 (C-3'), 98.1 (C-6), 93.4 (C-8).

MS (ESI): m/z (%) = 301 [M - H]- (100), 303 [M + H]+ (96), 325 [M + Na]+ (70), 627 [2M + Na]+ (18)

## Patentansprüche

1. Verfahren zur Herstellung einer Verbindung mit der folgenden Formel (1), 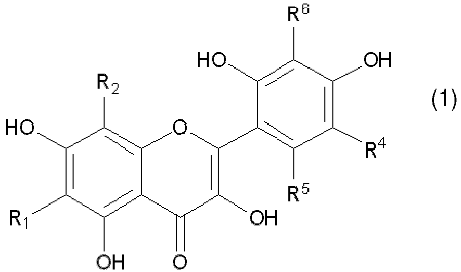 umfassend die Schritte:
i) Acetylierung einer Verbindung der folgenden Formel (3) 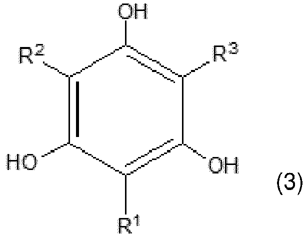 zur Bildung des Acetophenons der Formel (4), 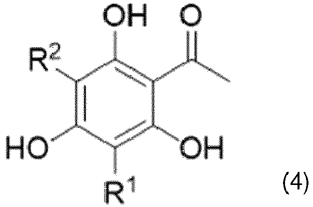
ii) Alkylierung des Acetophenons der Formel (4) dabei wird die Verbindung der Formel (6) gebildet 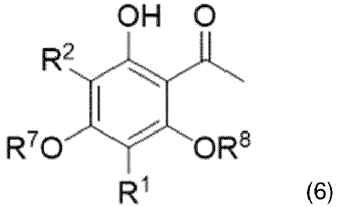
iii) Umsetzen des Acetophenons der Formel (6) mit einer Verbindung der folgenden Formel (7) 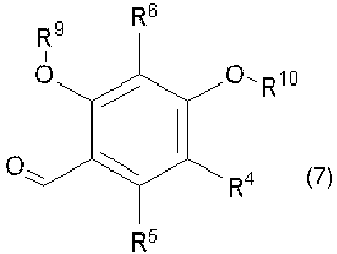 zur Bildung eines Flavonols der folgenden Formel (8), 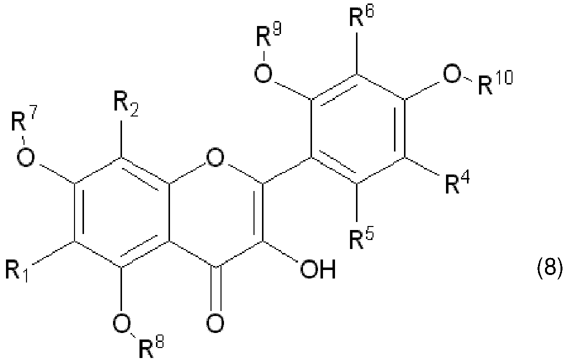 und
iv) Entfernung der an Sauerstoff gebundenen Schutzgruppen durch Dealkylierung, insbesondere Demethylierung, des Flavonols der Formel (8) zum Erhalt der Verbindung der Formel (1),
wobei
R¹, R², R³, R⁴, R⁵ und R⁶ unabhängig voneinander ein verzweigtes oder unverzweigtes C₁-C₈₋Alkyl, NO₂, SO₃H, NX₂, wobei X ein Ethyl- oder Methylrest oder Wasserstoff ist, CF₃ oder Wasserstoff sind, bevorzugt C₁-C₅-Alkyl oder Wasserstoff, insbesondere bevorzugt C₁-C₃-Alkyl oder Wasserstoff, und
R⁷, R⁸, R⁹ und R¹⁰ unabhängig voneinander eine Methyl-, Ethyl-, *tert*.-Butyl-, Benzyl-, Methoxymethyl-, *p*-Methoxybenzyl-, Benzyloxymethyl-, Triphenylmethyl-, Tetrahydropyranyl- , Allyl-, Trimethylsilyl-, Triethylsilyl-, Triisopropylsilyl- Gruppe sind und insbesondere R⁷, R⁸, R⁹ und R¹⁰ eine Methylgruppe bedeuten.

2. Verfahren gemäß Anspruch 1, welches den Verfahrensschritt i-a) umfasst und wobei R¹ bis R¹⁰ wie in Anspruch 1 definiert sind.

3. Verfahren gemäß Anspruch 2, wobei R³ Wasserstoff ist.

4. Verfahren gemäß einem der Ansprüche 1 bis 3, wobei R¹, R², R³, R⁴, R⁵ und R⁶ Wasserstoff sind.

5. Verfahren gemäß einem der Ansprüche 1 bis 4, wobei der Reaktionsschritt iii) in einer wässrigen Lösung oder wässrigen Mischung von Pyrrolidin, Piperidin, Morpholin oder Diethylamin erfolgt.

6. Verfahren nach einem der Ansprüche 1 bis 5, wobei Reaktionsschritt iii) in einem pH-Bereich von 12 - 14 durchgeführt wird.

7. Verfahren nach einem der Ansprüche 1 bis 6, wobei bei Reaktionschritt iii) zusätzlich Triplettgeneratoren eingesetzt werden.

8. Verfahren nach einem der Ansprüche 1 bis 7, wobei das Reaktionsgemisch iii) mit Wellenlängen von 350 bis 700 nm bestrahlt wird.

9. Verfahren gemäß einem der Ansprüche 1 bis 8, wobei in Schritt i) die Acetylierung mit Hilfe von Acetylchlorid in Anwesenheit einer Lewis-Säure und Dichlormethan oder Nitromethan erfolgt.

10. Verfahren zur Herstellung von Morin und Morinderivaten der Formel (1), welches die Umsetzung einer Verbindung der Formel (6) mit einer Verbindung der Formel (7) zu einer Verbindung der Formel (8) umfasst.
